(19) Europäisches Patentamt
European Patent Office
Office européen des brevets

(11) EP 3 598 115 A1

(12) EUROPEAN PATENT APPLICATION
published in accordance with Art. 153(4) EPC

(43) Date of publication:
22.01.2020 Bulletin 2020/04

(51) Int Cl.:
G01N 23/041 (2018.01)

(21) Application number: 18767370.2

(22) Date of filing: 08.03.2018

(86) International application number:
PCT/JP2018/008907

(87) International publication number:
WO 2018/168621 (20.09.2018 Gazette 2018/38)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 17.03.2017 JP 2017053046

(71) Applicant: Tohoku University
Aoba-ku
Sendai-shi
Miyagi 980-8577 (JP)

(72) Inventors:
• MOMOSE Atsushi
Sendai-shi
Miyagi 980-8577 (JP)
• IKEMATSU Katsumasa
Sendai-shi
Miyagi 980-8577 (JP)
• TAKANO Hidekazu
Sendai-shi
Miyagi 980-8577 (JP)

(74) Representative: Piotrowicz, Pawel Jan Andrzej et al
Venner Shipley LLP
Byron House
Cambridge Business Park
Cowley Road
Cambridge CB4 0WZ (GB)

(54) RADIOGRAPHIC IMAGE GENERATING DEVICE

(57) The present invention provides technology that can resolve a structure that is finer than the period of a periodic structure of a grating. A radiation source 1 is configured to irradiate radiation towards a grating section 2. A G1 grating 21 has a G1 periodic structure that forms concentrated sections 71 where intensity of the radiation 7 is concentrated, between the G1 grating 21 and a detector 3. The detector 3 detects radiation 7 that has passed through the grating section 2 as a moiré image. A sample translation section 4 translates the sample 10 in a direction along the periodic direction of the G1 periodic structure of the G1 grating 21, and so as to pass through the concentrated sections 71.

Fig. 1

Printed by Jouve, 75001 PARIS (FR)

## Description

Technical Field

The present invention relates to technology for observing the structure of a subject, utilizing wave properties of radiation that has passed through the subject, such as X rays.

Background Art

Radiation of high penetrating power, for example X-rays, is in widespread use as probes for visualizing the inside of a material in fields such as medical image diagnosis, non-destructive testing, security checks etc. Contrast of an X-ray perspective image depends on difference in X-ray attenuation factor, and a body that strongly absorbs X-rays is rendered as an X-ray shadow.
X-ray absorption power is stronger the more elements are contained that have a larger atomic number. Conversely, for a material that is composed of elements of small atomic number, it can be noted that it is also difficult to obtain contrast, and this is also a principle disadvantage with conventional X-ray perspective images. Accordingly, it has not been possible to obtain sufficient sensitivity with regard to soft biological tissue, organic material etc.
On the other hand, if the wave properties of X-rays are utilized, it is possible to realize a sensitivity increase of up to about three orders of magnitude compared to general conventional X-ray perspective images. Hereafter this will be referred to as an X-ray phase contrast method. If this technology is applied to observation of materials composed of light elements that do not absorb X-rays well (such as soft biological tissue, organic material etc.), then since examination becomes possible that was difficult with the conventional methods, such practical application is expected.
A method that uses a transmission grating is known as an approach for realizing a high sensitivity imaging method that utilizes the X-ray phase contrast method (refer to patent publications 1 and 2 below). This is a method for obtaining contrast that shows the structure of a subject by means of a phenomenon whereby an intensity pattern formed by a transmission grating that is being irradiated by X-rays on an X-ray detector varies due to slight refraction and dispersion of X-rays by a subject that is being irradiated with the same X-rays. With this method, it is possible to generally generate absorption images corresponding to conventional perspective images, refractive images showing magnitude of refraction of X-rays by the subject, and scattering images showing magnitude of scattering by the subject. In a case where grating period of a transmission grating that is used is minute, a detector is arranged at a position where the intensity pattern is strongly visible, taking into consideration fractional Talbot effect due to interference effect (so-called diffraction effect) caused by the grating. Also, in a case where the intensity pattern is so fine that it can not be resolved directly with a detector, one more transmission grating is arranged at that position and it is possible to visualize variations in the intensity pattern by creating a moiré. It should be noted that hereafter, a transmission grating will be called a G1 grating, or simply G1, while the second transmission grating will be called a G2 grating, or simply G2. A structure composed of G1 and G2 will be referred to as a Talbot interferometer.
In operating a Talbot interferometer, it is desirable for a spatial interference distance of radiation that is irradiated on G1 to be equal to the G1 period (period of the periodic structure of G1) or greater than that. This results in a need for radiation waves to be aligned, and with X-rays, for example, this is satisfied by using synchrotron radiation and a microfocus X-ray source. In particular, since a microfocus X-ray source is a radiation source that can be used in a laboratory it is worth noting when considering practical use.
However, output power of a microfocus X-ray source is generally limited, and so an exposure time of from a number of minutes to a few tens of minutes is normally required. An X-ray source that is generally used is higher power than a microfocus X-ray source, but in the first place a spatial coherence required in order to allow operation of an X-ray Talbot interferometer cannot be expected.
Therefore a Talbot-Lau interferometer having a third lattice (hereafter, G0) arranged in the vicinity of a general X-ray source is known. G0 behaves as a multislit. A single slit of G0 will be noted. An X-ray passing through this single slit makes a downstream Talbot interferometer (G1 and G2) function. Specifically, a single slit of G0 can be construed as virtually constituting a microfocus X ray source. Attention will focus on X-rays that pass through the next slit in G0. Similarly, the downstream Talbot interferometer is made to operate, but with intensity pattern due to G1 at a G2 position it is possible to adjust period of G0 such that the G1 pattern is offset by exactly one period (strictly speaking, an integral multiple of one period). By doing this, making phase contrast shooting high speed is realized using a conventional bright X-ray source that has low coherence while still generating moiré images using a down stream Talbot interferometer.
Accordingly, it can be recognized that a Talbot-Lau interferometer is a plurality of Talbot interferometers superimposed, and also that G0 is part of a radiation source. It is also possible to arrange only G0 and G1 close to the radiation source (radiation source without G0), and omit G2, and have a method of shooting the intensity pattern that has been expanded with a direct detector, and this is called a Lau interferometer.
With either configuration, direct use of an intensity pattern or a moire image that has been stored is scarce, and images that have been stored are processed by a given procedure using a computer, and it is possible to generate and use absorption images, refraction images and scattering images etc. A fringe scanning method is generally used for this purpose. A fringe scanning method is a method which either grating is translated in its periodic direction, a plurality of intensity patterns or moiré images are photographed, and image operations are carried out. More specifically, shooting is carried out by translating either grating by 1/M of its period, and image operations are carried out using M images that have been obtained by repeating this process M times. M is an integer of 3 or greater.

**Citation List**

Patent Literature

[Patent publication 1] International patent publication WO2004/058070
[Patent publication 2] USP 5812629

**SUMMARY OF THE INVENTION**

Problems To Be Solved By The Invention

With a conventional device that uses a Talbot-Lau interferometer or a Talbot interferometer, even if a fringe scanning method is used spatial resolution of images obtained is restricted by pattern period of a transmission grating used. This is because since pixel values of a detector can be given as integration values for at least one period of a lattice, it is essentially not possible to visualize a structure that is finer than the lattice period. Making the periodic structure of the lattice finer is effective in order to improve spatial resolution. However, as a lattice it is necessary to utilize a structure that has a wide radiation projection surface area and a high aspect ratio. This means that manufacturing a practical lattice that has a fine period is far from easy. Specifically, with a conventional device there is a problem in that it is difficult to resolve a structure that is finer than the period of the lattice pattern.

The present invention has been conceived in view of the above-described situation. A main object of the present invention is to provide technology that can resolve a structure that is finer than the period of a periodic structure of a lattice.

Means For Solving The Problems

The present invention can be expressed as inventions described in the following aspects.

(Aspect 1)

A radiographic image generating device for generating a radiographic image of a sample using a moiré image of radiation, comprising:

a radiation source, a grating section, a detector and a sample translation section, wherein

the radiation source is configured to irradiate radiation towards the grating section;
the grating section comprises at least a G1 grating;
the G1 grating has a G1 periodic structure that forms concentrated sections where radiation intensity is concentrated, between the G1 grating and the detector;
the detector is configured to detect the radiation that has passed through the lattice section as a moiré image; and
the sample translation section is configured to translate the sample so that it moves in a direction along the periodic direction of the G1 periodic structure, and passes through the concentrated sections.

(Aspect 2)

The radiographic image generating device of aspect 1, wherein, a width of the concentration sections, in a direction of the period of the G1 periodic structure, is less than or equal to 1/2 the period of the G1 periodic structure.

(Aspect 3)

The radiographic image generating device of aspect 1 or aspect 2, wherein the grating section further comprises a G2 grating, and wherein
the G2 grating comprises a G2 periodic structure that is substantially the same as the period of a self image of the G1 grating, that has been formed by the radiation that has passed through the G1 grating, that is at a position of the G2 grating; and
the detector detects the self image through the G2 grating as the moiré image.

(Aspect 4)

The radiographic image generating device of aspect 3, further comprising a grating translation section; wherein
the grating translation section is configured to translate the G2 grating a step of 1/k at a time with respect to the period of the G2 periodic structure, along the direction of the period of the G2 periodic structure, and wherein
k here is an integer of 3 or more.

(Aspect 5)

The radiographic image generating device of any one of aspect 1 to aspect 4, wherein the G1 periodic structure of the G1 grating has a cross-sectional shape, along the direction of the period of the G1 periodic structure, that is substantially a triangular wave shape.

(Aspect 6)

The radiographic image generating device of any one of aspect 1 to aspect 4, wherein the G1 periodic structure of the G1 grating has a cross-sectional shape, along the direction of the period of the G1 periodic structure, that is substantially a parabolic shape.

(Aspect 7)

A radiographic image generating method for generating a radiographic image of a sample using a radiographic moiré image, comprising:

a step of irradiating radiation towards a grating section that comprises at least a G1 grating having a G1 periodic structure;
a step of forming concentrated sections where radiation intensity is concentrated, between the G1 grating and a detector, by periodically changing intensity of the radiation using the G1 grating;
a step of detecting the radiation that has passed through the grating section as the moiré image using the detector; and
a step of translating the sample so that it moves in a direction along the periodic direction of the G1 periodic structure, and passes through the concentrated sections.

(Aspect 8)

A computer program for causing a computer to execute each of the steps described in aspect 7.
This computer program can be stored in a suitable storage medium (for example, electronic, optical, magnetic or magneto-optical storage medium). This computer program may also be transmitted by means of communication lines such as the Internet.

Effect of The Invention

According to the present invention it is possible to provide technology that can resolve a structure that is finer than the period of a periodic structure of a grating.

Brief Description of the Drawings

Fig. 1 is an explanatory drawing for describing the schematic structure of a radiographic image generating device of a first embodiment of the present invention.
Fig. 2 is an explanatory drawing for describing a Talbot carpet generated in the device of Fig. 1.
Fig. 3 is a graph showing radiation intensity in a case where the Talbot carpet of Fig. 2 has been cut in the direction of the grating period, for concentrated sections 71 (black arrows) where intensity is concentrated, with the horizontal axis representing distance in the periodic direction of the grating, and the vertical axis representing radiation intensity.
Fig. 4 is a flowchart showing an outline of an image generating method using the device of Fig. 1.
Fig. 5 is an explanatory drawing for describing a procedure for fringe scanning and sample scanning with the image generating method of Fig. 4, with the horizontal direction representing movement distance of the grating, and the vertical direction representing movement distance of the sample.
Fig. 6 is an explanatory drawing for describing a procedure for fringe scanning and sample scanning with the image generating method of Fig. 4.
Fig. 7 is an explanatory drawing for describing a procedure for image construction with the image generating method of Fig. 4.
Fig. 8 is an explanatory drawing for describing a G1 grating used in a radiographic image generating device of a second embodiment of the present invention.
Fig. 9 is a graph showing radiation intensity in a case where the Talbot carpet of Fig. 8 has been cut in the direction of the grating period, for concentrated sections 71 (black arrows) where intensity is concentrated, with the horizontal axis representing distance in the periodic direction of the grating, and the vertical axis representing radiation intensity.
Fig. 10 is an explanatory drawing for describing a G2 grating used in the radiographic image generating device of Fig. 8.
Fig. 11(a) is an explanatory drawing for describing a G1 grating used in a radiographic image generating device of a third embodiment of the present invention, and Fig. 11(b) is an explanatory drawing showing a comparative example. It should be noted that in these drawings the horizontal axis represents distance (m).

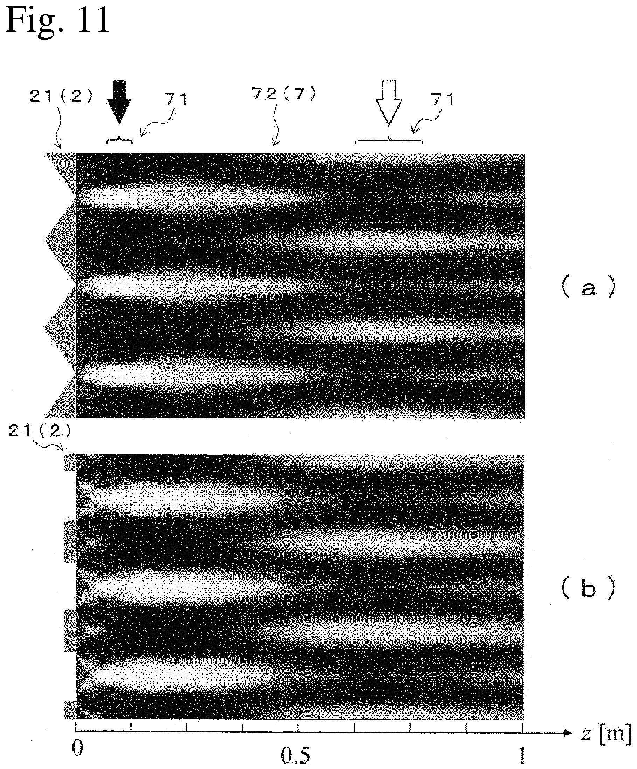

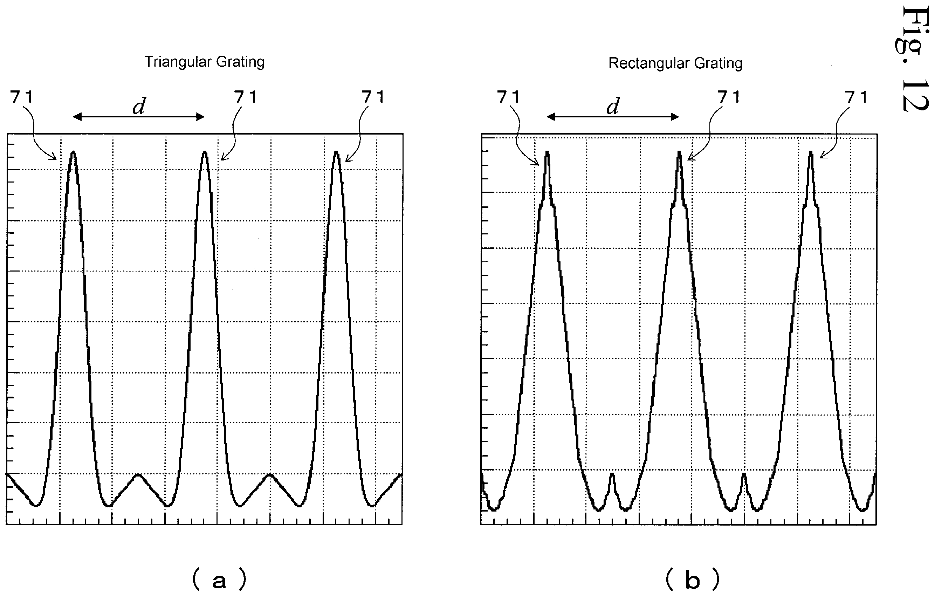

Fig. 12(a) is a graph showing radiation intensity in a case where the Talbot carpet that was generated by the grating of Fig. 11(a) has been cut in the direction of the grating period, for positions of black arrows, with the horizontal axis representing distance in the periodic direction of the grating, and the vertical axis representing radiation intensity. Fig. 12(b) is a graph showing radiation intensity in a case where the Talbot carpet that was generated by the grating of Fig. 11(b) has been cut in the direction of the grating period, for positions of black arrows, with the horizontal axis representing distance in the periodic direction of the grating, and the vertical axis representing radiation intensity.
Fig. 13a is an explanatory drawing for describing a G1 grating used in a radiographic image generating device of a fourth embodiment of the present invention, and is an explanatory drawing showing method of arrangement by tilting a rectangular grating as shown in Fig. 13(a) that has an equivalent action to the triangular grating of Fig. 13(b).

Fig. 14 is an explanatory drawing for describing a procedure for fringe scanning and sample scanning with a radiographic image generating method of a fifth embodiment of the present invention, with the horizontal direction representing movement distance of the grating, and the vertical direction representing movement distance of the sample.

**Description of the Embodiments**

(Structure of First Embodiment)

In the following, a radiographic image generating device (hereafter sometimes simply abbreviated to "device") of a first embodiment of the present invention will be described with reference to the attached drawings. This device is for generating a radiographic image (for example, either or all of an absorption image, a refraction image, or a scattering image) of a sample using moiré images of radiation. This device targets either an organism or object other than an organism as a sample. Also, this device can be used in medical applications or non-medical applications. As an application in non-medical fields, it is possible to exemplify the examination of foodstuffs, industrial parts, or industrial products, but these are not limiting.

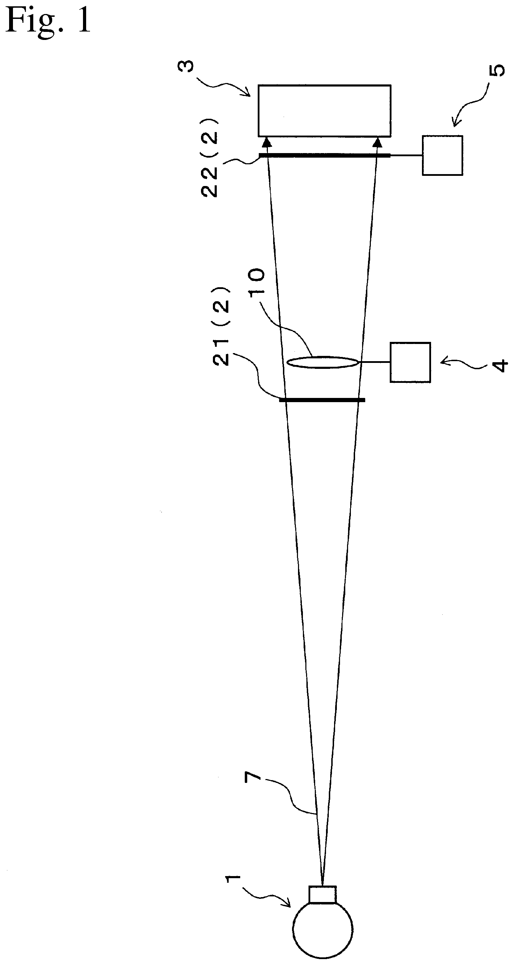

The device of this embodiment comprises a radiation source 1, a grating section 2, a detector 3, and a sample translation section 4 as basic elements (refer to Fig. 1). The device of this embodiment is additionally provided with a grating translation section 5.

(Radiation Source)

The radiation source 1 is configured to irradiate radiation 7 towards the grating section 2. As the radiation source 1 of this embodiment, a source generates radiation that has a spatial coherence length that is sufficient to make a Talbot interferometer operate using the grating section 2. This means there is a need for radiation waves to be aligned, and with X-rays as radiation, for example, this is satisfied by using synchrotron radiation and a microfocus X-ray source as the radiation source 1.

(Grating Section)

The grating section 2 comprises a G1 grating 21 and a G2 grating 22. Specifically, the grating section 2 of this embodiment constitutes a so called Talbot interferometer.

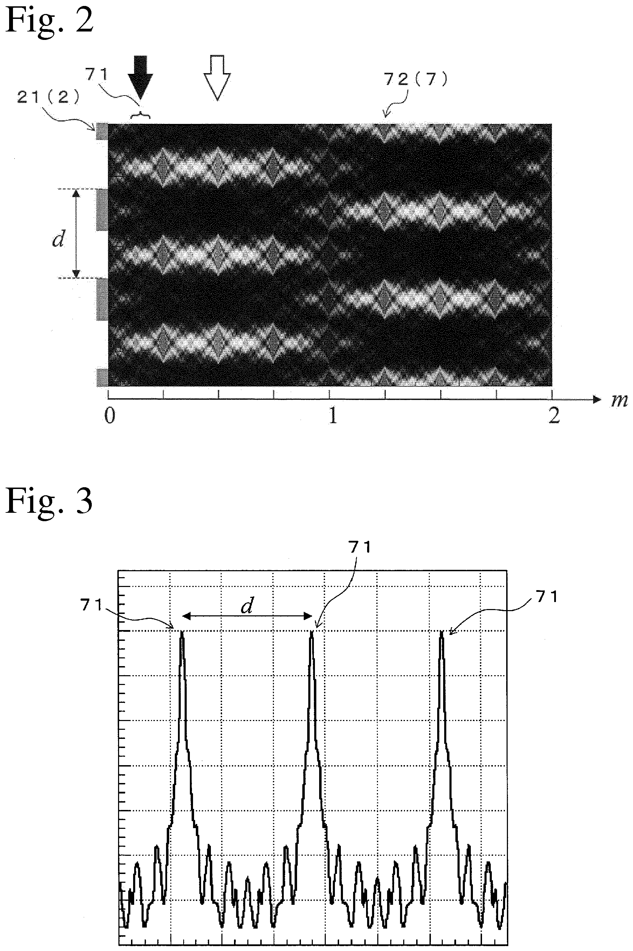

The G1 grating 21 has a G1 periodic structure that forms concentrated sections 71 (refer to Fig. 2 and Fig. 3) where intensity of the radiation 7 is concentrated, between the G1 grating 21 and the detector 3. In more detail, looking from the radiation source 1, the concentrated sections 71 are formed behind the G1 grating 21 and in front of the G2 grating 22.

Width of the concentrated sections 71 of the illustrated example in the periodic direction (the vertical direction in Fig. 2) of the G1 periodic structure is made 1/2 or less the period of the G1 periodic structure.

The G2 grating 22 is provided with a G2 periodic structure of substantially the same period that a self image of the G1 grating 21 that has been formed by the radiation 7 that has passed through the G1 grating 21 has, at the position of the G2 grating 22. The G2 grating 22 of this example can be moved, as will be described later, using the grating translation section 5.

(Detector)

The detector 3 is configured to detect radiation 7 that has passed through the grating section 2 as a moiré image. In more detail, the detector 3 of this embodiment is configured to detect a self image of the G1 grating 21, via the G2 grating 22, as a moiré image. Further, the detector 3 is configured so that it is possible to generate a desired radiographic image by performing normal processing for phase imaging of a fringe scanning method, using k (here, k is an integer of 3 or more) moiré images that have been obtained in accordance with translation of the G2 grating 22. A detector that is the same as a conventional detector may be used as the detector 3, and so more detailed description is omitted.

(Sample Translation Section)

The sample translation section 4 is configured to translate the sample 10 in a direction along the periodic direction of the G1 periodic structure of the G1 grating 21 (the vertical direction in Fig. 2), and so as to pass through the concentrated sections 71. The sample translation section 4 of this embodiment is configured so that it is possible to translate the G1 grating 21 step of $1/N$ (here N is an integer of 2 or more) of the G1 grating period d (refer to Fig. 2) at a time. The sample translation section 4 is not particularly restricted, but it is possible to use a linear motion mechanism or Piezoelectric element that can translate the sample for every specified step.

(Grating Translation Section)

The grating translation section 5 is configured to move the G2 grating 22 by a step of $1/k$ (here k is an integer of 3 or more) at a time with respect to period of the G2 periodic structure along the direction of the period of the G2 periodic structure. The grating translation section 5 is not particularly restricted, but it is possible to use a linear motion mechanism or Piezoelectric element that can translate the grating for every designated step.

(Theoretical Description of Radiographic Image Generating Method)

The principles of radiographic image generation for this embodiment will be described in the following. A specific method for image generation will be described later.

Downstream of the G1 grating 21 a characteristic intensity pattern appears dependent on a pattern shape of the G1 grating 21 (specifically, the G1 periodic structure), as a result of various diffracted waves that have passed through this grating interfering with each other, and this pattern changes in accordance with distance from the G1 grating 21. In this specification a representation of the appearance of this change is called a Talbot carpet 72. If the G1 grating 21 is made a rectangular π/2 phase grating, a Talbot carpet 72 that this grating makes is shown in Fig. 2. In this drawing the horizontal axis m and distance z from the G1 grating 21 have a relationship of

$$z = m \frac{d^2}{\lambda} \cdot \frac{R}{R - m \frac{d^2}{\lambda}} .$$

It should be noted that in this equation d is period of the G1 periodic structure of the G1 grating 21, λ is the wavelength of X-rays, and R is distance from the radiation source 1 to the G1 grating 21.

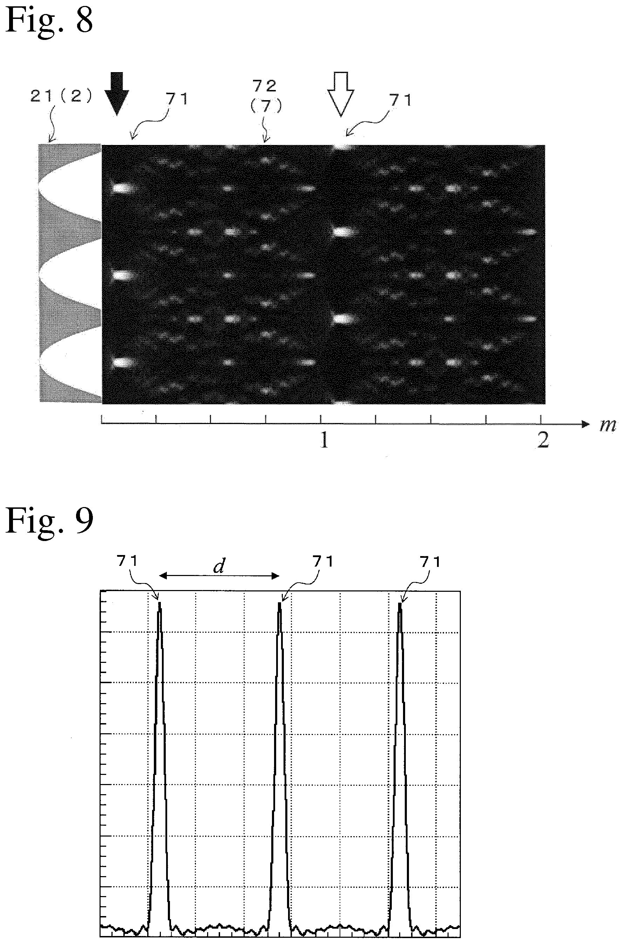

The G2 grating 22 is generally placed at a position (for example, position shown by white arrows in Fig. 2) where m becomes half an integer (for example 0.5). It should be noted that effective dimensions of the Talbot carpet of Fig. 2 (the same applies to Fig. 8 which will be described later) are that period d is a few μm, and a distance corresponding to m=0.5 is a few tens of cm, and a figure that is very long crosswise in actual scale is displayed here with a ratio changed. Also, a grating pattern of each grating (periodic structure) is effectively formed on a substrate, but the substrate is not shown in the drawings.

With this example, a moiré image is recorded by the detector 3 that has been placed behind the G2 grating 22. A plurality of moiré images are measured while translating one of the G1 grating 21 and the G2 grating 22, and it is possible to generate absorption images, refraction images, and scattering images by applying computer calculation processing (fringe scanning method).

Spatial resolution of shooting moiré images is dependent on focus size of the radiation source 1 and resolution of the detector 3, but even if these are ideal it will not be possible to exceed the limit that is determined by two-times the period of the G1 grating 21. In order to realize an even higher spatial resolution by exceeding this limit, a method of determining position where the sample 10 is arranged, and acquiring a plurality of images by moving the sample 10 more finely than the grating period, will be described in the following.

(radiographic image generating method of the first embodiment)

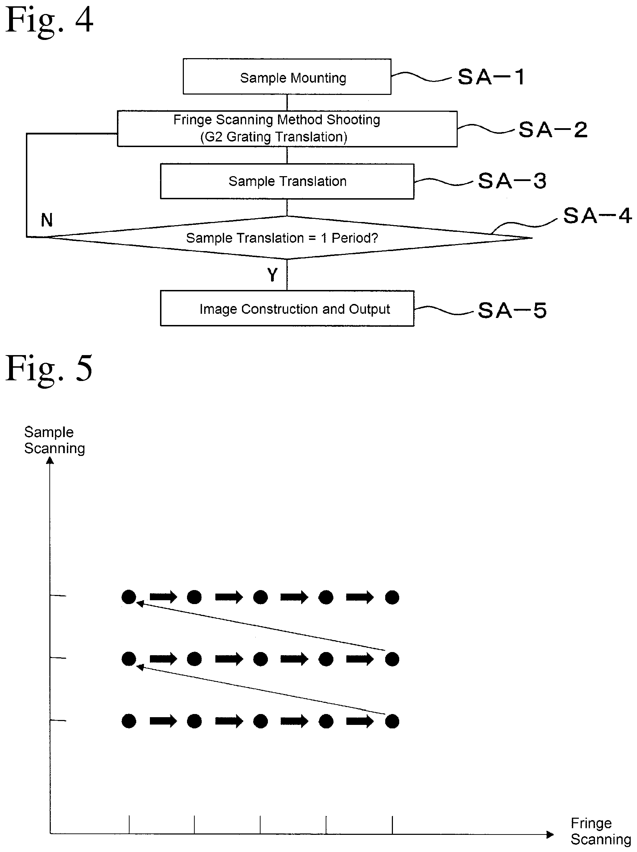

Next, a radiographic image generating method that uses the previously described device will be described with further reference to Fig. 4.

(Step SA-1 of Fig. 4)

First, the sample 10 is arranged at a position where the concentrated sections 71 should be formed, between the G1 grating 21 and the G2 grating 22, or close to that position. For example, with the example of Fig. 2 the sample 10 is arranged at a position shown by a black arrow in the drawing (m ≈ 0.2). Here, the position where the sample 10 is arranged is preferably a position where size of the concentrated sections 71 (size in the periodic direction of the G1 grating) becomes small.

(Step SA-2 of Fig. 4)

Next, similarly to the related art, shooting is performed using a fringe scanning method. Specifically, for example, the G2 grating 22 that has been arranged at the position shown by the white arrow (m≒0.5) in the drawing is translated by the grating translation section 5, and is relatively moved with respect to the G1 grating 21. Here, movement steps of the G2 grating 22 are made 1/k of the grading period of the G2 grating 22 (k is an integer of 3 or more). On the other hand, the detector 3 obtains moiré images of radiation that has been irradiated from the radiation source 1 each time the G2 grating 22 moves by one step. Using moiré images that have been taken in this way it is possible to generate a radiographic image, similarly to the related art.

A relationship between grating translation steps and shooting timing is shown in Fig. 5. In Fig. 5 the horizontal axis represents grating translation distance, the vertical axis represents sample translation distance (described later), and black circles represent shooting timing. With this example k=5 has been set, but is not thus limited.

(Steps SA-3 to SA-4 in Fig. 4)

Next, with this embodiment, the sample 10 is moved for every step of 1/N of the grating period of the G1 grating (N is an integer of 2 or more), by the sample translation section 4. When the sample translation distance is less than a single period, processing returns to previously described step SA-2, and shooting for a fringe scanning method is performed again. Specifically, as shown in Fig. 5, the sequence of shooting using a fringe scanning method→sample translation→shooting using a fringe scanning method→ ... is repeated. With this example N=3 has been set, but is not thus limited.

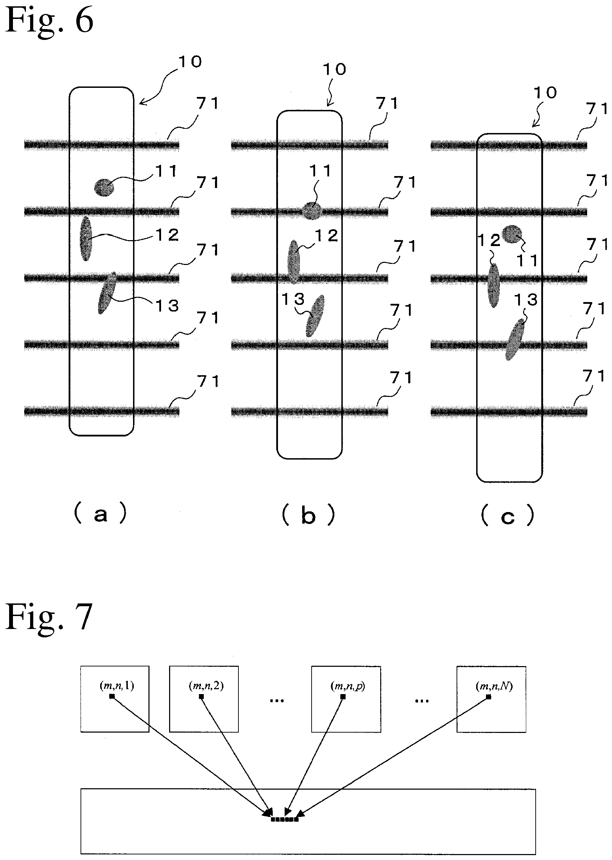

A relationship between translation of the sample 10 and shooting timing will be further described with reference to Fig. 6. It is assumed that microstructures 11 to 13 exist within this sample 10. Also, with this example, the concentrated sections 71 of the radiation 7 are illustrated for 5 periods in the periodic direction of the G1 grating 21 (the vertical direction in Fig. 6). First, shooting for a fringe scanning method is performed at an initial position of the sample 10 (Fig. 6(a)). Here, noting the structure 11 within the sample 10, since there is no irradiation of radiation to the structure 11, effects of the structure 11 (for example, absorption, refraction or scattering) do not appear in a moiré image that has been taken by the detector 3. Accordingly, information on the structure 11 is not contained in the moiré image. Next, the sample 10 is moved by a single step (Fig. 6(b)). Once the sample 10 has been moved, radiation is irradiated to the structure 11 at a concentrated section 71. If radiation has been irradiated, the effect of the structure 11 appears in the moiré image that has been acquired by the detector 3, and as a result it is possible to resolve the structure 11. The same also applies to the structure 12. A state where the sample 10 has been moved further is shown in Fig. 6(c).

In this way, according to this embodiment, by translating the sample 10 there is the advantage that it is possible to resolve a structure that is smaller than the period of G1 grating 21.

(Step SA-5 of Fig. 4)

When sample translation has reached one period, a high resolution image is constructed using moiré images that have been acquired from the detector 3. A method for this image construction will be described with reference to Fig. 7. In Fig. 7, a two dimensional pixel position of the detector 3 is represented by (m, n), and a number of steps of sample translation is represented by p. A pixel value for a specified step p is then made (m, n, p). Here, p=1, 2, ... N. By performing a fringe scanning method at respective positions p, N images are acquired as absorption images, refraction images, and scattering images. It is possible to acquire a new high resolution image by using these images in which pixel values (m, n, p) of respective images are aligned.

(Second Embodiment)

Next, a device of a second embodiment of the present invention will be described with reference to Fig. 8. In the description of this second embodiment, structural elements that are basically common to the first embodiment described above will be assigned the same reference numerals, and redundant description be avoided.

With the device of the second embodiment, a grating that has a cross-section formed into a parabolic shape is used as the G1 grating 21. Specifically, this G1 grating 21 is configured so that a paraboloid that extends in a depth direction of the drawing sheet of Fig. 8 is formed. In Fig. 8, the open section of this paraboloid faces towards a downstream side of the radiation direction of the radiation 7, but it may also face towards the upstream side.

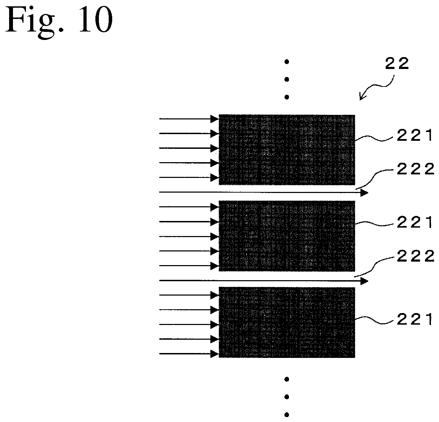

According to the G1 grating of this second embodiment, it is possible to cause radiation intensity to concentrate more strongly (that is, with a narrower half-power width) at the concentrated sections 71, as shown in Fig. 9. With the example of Fig. 8, respective concentrated sections 71 are formed at the black arrow portions and white arrow portions within the drawing. With the second embodiment, a sample is arranged at the concentrated sections 71 shown by the black arrow, and the G2 grating 22 is arranged at concentrated sections 71 shown by the white arrow. As the G2 grating 22 of this embodiment, it is preferable to use a structure where radiation transmissible sections 222 have been narrowed relative to radiation shielding sections 221, as shown in Fig. 10. Width of the radiation transmissible sections 222 is made substantially the same as width of the concentrated sections 71. If this type of structure is adopted, there is the advantage that it becomes possible to resolve the even finer structures of the sample 10.

It should be noted that Fig. 9 is a graph of results that have been acquired by having calculated on the assumption that the spatial coherence of the radiation 7 is perfect, but if spatial coherence of the radiation 7 becomes moderately lower than that, a Talbot carpet will become blurred moving away from the G1 grating. Specifically, while change is slight with spread in concentrated sections 71 where the sample is arranged, it is possible to adjust spatial coherence of the radiation 7 so as to widen concentrated sections 71 at white arrow positions where the G2 grating 22 is arranged. In this way, the G2 grating 22 generally does not have a structure that is difficult to manufacture, such as with the radiation transmissible sections 222 relatively narrow with respect to the radiation shielding sections 221, and it is possible to adopt a structure where the radiation shielding sections 221 and the radiation transmissible sections 222 are substantially equal. Since a Talbot interferometer structure where the radiation shielding sections 221 and the radiation transmissible sections 222 are substantially equal has generally been used, if such a device is used there is the advantage that it is possible to reduce manufacturing cost of the grating.

Since other structures and advantages of the second embodiment are basically the same as those of the previously described first embodiment, more detailed description will be omitted.

(Third Embodiment)

Next, a device of a third embodiment of the present invention will be described with reference to Fig. 11. In the description of this third embodiment, structural elements that are basically common to the first embodiment described above will be assigned the same reference numerals, and redundant description be avoided.

With each of the above-described embodiments, monochromatic X-rays have been provided as the radiation 7. Conversely, with the device of this third embodiment heterogeneous X rays are used as the radiation 7. Also, a grating that has a cross-section formed in a triangular wave shape is used as the G1 grating 21, as shown in Fig. 11(a). Here, as simulation conditions, period of the G1 grading is made 5 microns, and spectral range of the x-rays was calculated as ±10 keV with a center of 25 keV. It should be noted that a triangular grating is made a shape that gives a phase difference of $\pi$ for X-rays of 25 keV, at the vertex.

According to the G1 grating of this third embodiment, it is possible to cause radiation intensity to concentrate at the concentrated sections 71, as shown in Fig. 11(a) and Fig. 12(a). For comparison, results of having irradiated radiation under the same conditions with a cross section of the G1 grating 21 made rectangular are shown in Fig. 11(b) and Fig. 12(b). Here also, as simulation conditions, period of the G1 grating is made 5 microns, and spectral range of the x-rays was calculated as ±10 keV with a center of 25 keV. It should be noted that the rectangular grating is made a shape that gives a phase difference of $\pi/2$ for 25 keV X-rays. As will be understood from these results, by making the cross-sectional shape of the G1 grating 21 triangular, it is possible to increase the degree of concentration of radiation at the concentrated sections 71.

Since other structures and advantages of the third embodiment are basically the same as those of the previously described first embodiment, more detailed description will be omitted.

(Fourth Embodiment)

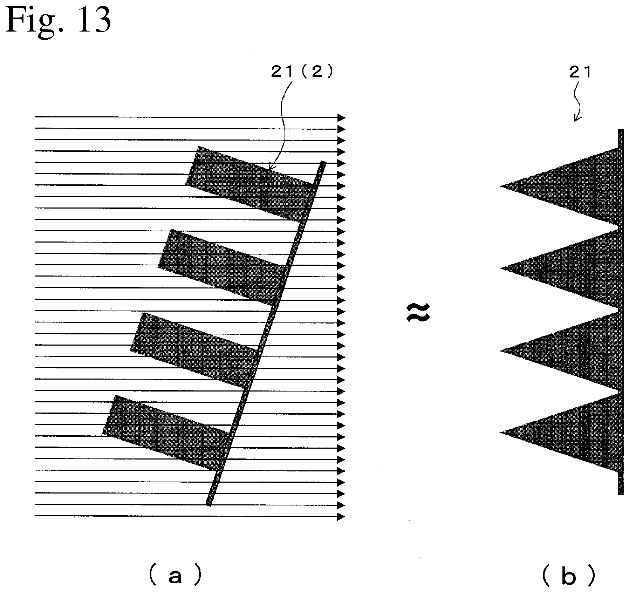

Next, a device of a fourth embodiment of the present invention will be described with reference to Fig. 13. In the description of this fourth embodiment, structural elements that are basically common to the third embodiment described above will be assigned the same reference numerals, and redundant description be avoided.

In the previously described third embodiment a G1 grating 21 having a triangular wave cross section was used, but with the fourth embodiment a G1 grating 21 having a structure where a rectangular cross-section grating is arranged in an inclined manner, is used (Fig. 13(a)). If this type of structure is adopted, then it is possible to form concentrated sections 71 that are equivalent to a triangular wave cross-section (Fig. 13(b)), even with a grating having a rectangular cross-section. Accordingly, with the fourth embodiment there is the advantage that it is possible to reduce manufacturing cost of the grating.

Since other structures and advantages of the fourth embodiment are basically the same as those of the previously described third embodiment, more detailed description will be omitted.

(Fifth Embodiment)

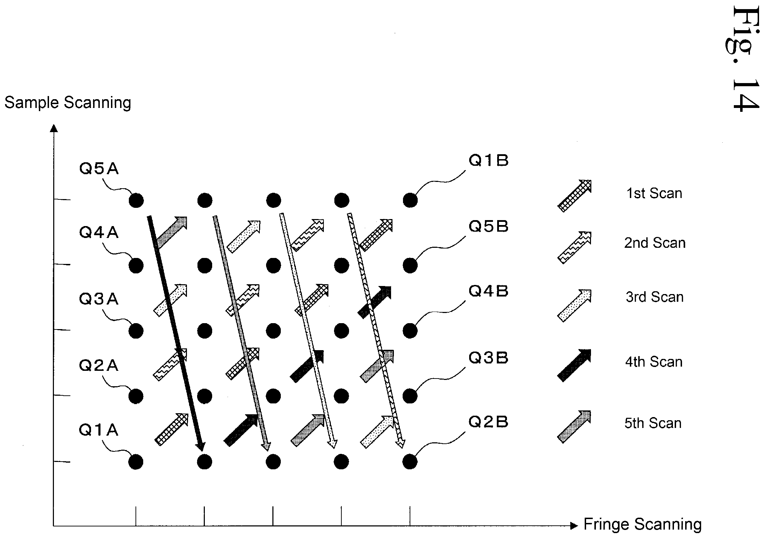

Next, a device of a fifth embodiment of the present invention will be described with reference to Fig. 14. In the description of this fifth embodiment, structural elements that are basically common to the first embodiment described above will be assigned the same reference numerals, and redundant description be avoided.

In each of the above-described embodiments, the G2 grating 22 was moved a specified distance at a time in order to implement a fringe scanning method. Conversely, with this fifth embodiment, a fringe scanning method is performed by moving the G1 grating 21 a specified distance at a time. In a case where the G1 grating 21 has been moved, it is also possible to move the sample 10 and the G1 grating 21 in synchronism by 1/k of the G2 grating period at a time. If this approach is adopted, then since there is no relative change in a positional relationship between the G1 grating 21 and the sample 10, it is possible to perform the same processing as in the previously described embodiments.

However, with this embodiment a method is adopted where the G1 grating 21 is moved by 1/k of the G1 grating period at a time, without moving the sample 10. In this case, since a positional relationship between the G1 grating 21 and the sample 10 changes in accordance with movement of the G1 grating 21, it is necessary to exercise care in the handling of images that have been detected by the detector 3. It is also necessary to make the number of steps of fringe scanning and the number of steps of sample scanning equal.

This processing will be described in more detail using Fig. 14. First, in a first scan, as shown in Fig. 14, shooting is performed for every step of 1/k, in accordance with movement of the G1 grating 21 from a start position Q1A to an end position Q1B. With this example the number of steps is five. After completion of the first scam, the sample 10 is moved by a single step of 1/k by the sample translation section 4. Shooting is then performed for every step in accordance with movement of the G1 grating 21 from a start position Q2A to an end position Q2B. Subsequently, in a similar manner, shooting is performed for from start position Q3A to end position Q3B, from start position Q4A to end position Q4B, and from start position Q5A to end position Q5B. In this way it is possible to acquire shooting data at each position, as shown by the black circles in Fig. 14. In generation of a radiographic image later, it is possible to use a single row along the fringe scanning direction (a single row along the horizontal axis in the case of Fig. 14) as fringe scanning data, and a single row just above that row as fringe scanning data after the sample has been moved by a single step. In this way it is possible to realize the same fringe scanning method as in the example of Fig. 5.

It should be noted that the descriptions of the above-described embodiments and practical example are merely examples, and do not show the essential structure of the present invention. The structure of each part is not limited to the above description as long as it falls within the scope of the present invention.

For example, with the previously described embodiment an x-ray source has been used as the radiation source 1, but it is also possible to use other radiation that has transmissivity with respect to the sample, for example, a neutron source. Obviously, in this case, the detector is capable of detecting the radiation source that is used.

Also, a one-dimensional grating is used as the grating with each of the above-described embodiments. Specifically, with the example of Fig. 2, for example, the grating extends in a direction that is orthogonal to the drawing sheet, and there is no change in intensity on a Talbot carpet 72 in the direction that is orthogonal to the drawing sheet. However, it is also possible to have a configuration where a two dimensional grating is used as the grating, and the sample is also translated two dimensionally. If this approach is taken, it is possible to achieve high resolution two dimensionally, both horizontally and vertically.

Further, with each of the above-described embodiments, a radiographic image is generated using a fringe scanning method, but it is also possible to use moiré images themselves as radiographic images. In this case, acquisition of moiré images corresponds to the generation of a radiographic image of the present invention.

Also, although the grating section 2 that comprises a G1 grating and a G2 grating has been used with each of the above-described embodiments, it is also possible to use a grating section 2 to which a G0 grating, constituting a Talbot-Lau interferometer, has been added. It is also possible to use a grating section 2 that constitutes a Lau interferometer from which the G2 grating has been omitted.

Description of the Numerals

    1 radiation source
    2 grating section
    21 G1 grating
    22 G2 grating
    221 radiation shielding sections
    222 radiation transmissible sections
    3 detector
    4 sample translation section
    5 grating translation section

    7 radiation
    71 concentrated sections
    72 Talbot carpet
    10 sample
    11 - 13 structures within sample

Claims

1.  A radiographic image generating device for generating a radiographic image of a sample using a moiré image of radiation, comprising:

    a radiation source, a grating section, a detector and a sample translation section,
    wherein
    the radiation source is configured to irradiate radiation towards the grating section;
    the grating section comprises at least a G1 grating;
    the G1 grating has a G1 periodic structure that forms concentrated sections where radiation intensity is concentrated, between the G1 grating and the detector;
    the detector is configured to detect the radiation that has passed through the grating section as a moiré image; and
    the sample translation section is configured to translate the sample so that it moves in a direction along the periodic direction of the G1 periodic structure, and passes through the concentration sections.

2.  The radiographic image generating device of claim 1, wherein, width of the concentration sections, in a direction of the period of the G1 periodic structure, is less than or equal to 1/2 the period of the G1 periodic structure.

3.  The radiographic image generating device of claim 1 or claim 2, wherein the grating section further comprises a G2 grating, and wherein:

    the G2 grating comprises a G2 periodic structure that is substantially the same as the period of a self image of the G1 grating, that has been formed by the radiation that has passed through the G1 grating, that is at a position of the G2 grating; and
    the detector detects the self image via the G2 grating as the moiré image.

4.  The radiographic image generating device of claim 3, further comprising a grating translation section; wherein
    the grating translation section is configured to move the G2 grating a step of 1/k at a time with respect to the period of the G2 periodic structure, along the direction of the period of the G2 periodic structure, and wherein

k here is an integer of 3 or more.

5. The radiographic image generating device of any one of claim 1 to claim 4, wherein the G1 periodic structure of the G1 grating has a cross-sectional shape, along the direction of the period of the G1 periodic structure, that is substantially a triangular wave shape.

6. The radiographic image generating device of any one of claim 1 to claim 4, wherein the G1 periodic structure of the G1 grating has a cross-sectional shape, along the direction of the period of the G1 periodic structure, that is substantially a parabolic shape.

7. A radiographic image generating method for generating a radiographic image of a sample using a moiré image of radiation, comprising:

a step of irradiating radiation towards a grating section that comprises at least a G1 grating having a G1 periodic structure;
a step of forming concentrated sections where radiation intensity is concentrated, between the G1 grating and a detector, by periodically changing intensity of the radiation using the G1 grating;
a step of detecting the radiation that has passed through the grating section as the moiré image using the detector; and
a step of translating the sample so that it moves in a direction along the periodic direction of the G1 periodic structure, and passes through the concentrated sections.

8. A computer program for causing a computer to execute each of the steps described in claim 7.

Fig. 1

Fig. 2

Fig. 3

# Fig. 4

Sample Mounting — SA-1

Fringe Scanning Method Shooting (G2 Grating Translation) — SA-2

Sample Translation — SA-3

Sample Translation = 1 Period? — SA-4

N

Y

Image Construction and Output — SA-5

# Fig. 5

Sample Scanning

Fringe Scanning

# Fig. 6

( a )　　　　　( b )　　　　　( c )

# Fig. 7

Fig. 8

Fig. 9

Fig. 10

## Fig. 11

Fig. 12

(b) Rectangular Grating

(a) Triangular Grating

Fig. 13

21（2）

21

≈

（a）

（b）

# Fig. 14

# EP 3 598 115 A1

## INTERNATIONAL SEARCH REPORT

| International application No. |
|---|
| PCT/JP2018/008907 |

**A. CLASSIFICATION OF SUBJECT MATTER**
Int. Cl. G01N23/041(2018.01)i

According to International Patent Classification (IPC) or to both national classification and IPC

**B. FIELDS SEARCHED**

Minimum documentation searched (classification system followed by classification symbols)
Int. Cl. G01N23/041, G01N23/04, G01N23/20, A61B6/00

Documentation searched other than minimum documentation to the extent that such documents are included in the fields searched
Published examined utility model applications of Japan      1922-1996
Published unexamined utility model applications of Japan    1971-2018
Registered utility model specifications of Japan            1996-2018
Published registered utility model applications of Japan    1994-2018

Electronic data base consulted during the international search (name of data base and, where practicable, search terms used)
Google

**C. DOCUMENTS CONSIDERED TO BE RELEVANT**

| Category* | Citation of document, with indication, where appropriate, of the relevant passages | Relevant to claim No. |
|---|---|---|
| X<br>A | JP 2014-521101 A (UCL BUSINESS PLC) 25 August 2014, paragraphs [0017], [0047], fig. 2, 3 & US 2014/0233699 A1, paragraphs [0028], [0057], fig. 2, 3 & WO 2013/011317 A1 | 1, 7, 8<br>2-6 |
| A | JP 2012-13557 A (CANON INC.) 19 January 2012 (Family: none) | 1-8 |
| A | JP 2017-44603 A (TOHOKU UNIVERSITY) 02 March 2017 & WO 2017/033854 A1 | 1-8 |
| A | WO 2015/064723 A1 (TOHOKU UNIVERSITY) 07 May 2015 & US 2016/0252470 A1 & EP 3064930 A1 & CN 105637351 A & KR 10-2016-0054609 A | 1-8 |

☒ Further documents are listed in the continuation of Box C.     ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 21.05.2018 | 29.05.2018 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

21

**INTERNATIONAL SEARCH REPORT**

| | International application No. |
|---|---|
| | PCT/JP2018/008907 |

C (Continuation).    DOCUMENTS CONSIDERED TO BE RELEVANT

| Category* | Citation of document, with indication, where appropriate, of the relevant passages | Relevant to claim No. |
|---|---|---|
| A | Bachche, S. et al., X-ray phase scanner using Talbot-Lau interferometry for non-destructive testing - II, 第63回応用物理学会春季学術講演会 講演予稿集, 2016, 05-024 (Proceedings of the 63rd JSAP Spring Meeting) | 1-8 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004058070 A
- US 5812629 A